# FASCICULE DE BREVET EUROPEEN

(11) **EP 0 692 240 B1**
(45) Date de publication et mention de la délivrance du brevet: **03.01.2001**
(21) Numéro de dépôt: 95401329.8
(22) Date de dépôt: 08.06.1995
(51) Int. Cl.: A61K 7/00, A61K 7/50, A61K 7/48, A61K 7/02

(54) **Composition nettoyante solide pour la peau contenant un agent structurant particulaire**
Eine teilchenförmige strukturgebende Substanz enthaltendes festes Körperreinigungsmittel
Solid skin cleaning composition containing a particulated structurant

(30) Priorité: 11.07.1994 FR 9408565
(43) Date de publication de la demande: 17.01.1996
(73) Titulaire: L'OREAL, S.A., 75008 Paris (FR)
(72) Inventeur: Sebillotte-Arnaud, Laurence, F-94000 Creteil (FR)
(74) Mandataire: Dodin, Catherine

(56) Documents cités:
- EP-A- 0 252 463
- EP-A- 0 254 612
- EP-A- 0 486 394
- EP-A- 0 614 656
- WO-A-94/14402
- DE-A- 2 521 003
- US-A- 3 645 904

## Description

La présente invention a pour objet une nouvelle composition nettoyante pour la peau ayant un aspect de solide déformable. Du fait de son extrème douceur, cette composition peut être appliquée aussi bien sur le visage que sur le corps.

L'invention se rapporte aussi à un procédé de traitement cosmétique pour nettoyer la peau.

L'invention se rapporte encore à l'utilisation d'un agent structurant particulier dans une composition nettoyante pour la peau.

Les compositions nettoyantes pour la peau se présentent habituellement sous forme de pains solides comme les savons, ou sous forme de liquides plus ou moins visqueux.

Lorsque l'on utilise un savon, on se sert du savon entier lors de chaque utilisation, si bien que, peu à peu, il devient mou à force d'avoir été en contact avec l'eau et vieillit mal. A la fin, il est fréquent qu'il se casse et que l'utilisateur se trouve avec des petits bouts de savon difficiles à utiliser. De plus, le savon mouillé est glissant, ce qui rend son emploi malaisé, notamment pour les jeunes enfants. De ce fait, il est devenu courant d'utiliser des liquides nettoyants à la place des savons. Mais, plus les compositions sont liquides, plus il est difficile de les doser, car elles ont tendance à s'échapper entre les doigts. Par ailleurs, plus elles sont liquides, plus elles ont tendance à s'échapper de leur conditionnement.

De façon simplifiée, une composition nettoyante liquide comprend un ou plusieurs tensioactifs dans un support cosmétiquement acceptable contenant de l'eau.

Après chaque utilisation d'une composition nettoyante liquide, il est nécessaire de rincer la peau nettoyée. Malheureusement, un grand nombre de compositions liquides présente l'inconvénient d'être difficile à rincer et/ou de laisser des traces de produit sur la peau.

Par ailleurs, les utilisateurs recherchent, de plus en plus, de nouvelles textures et de nouveaux concepts de produits.

La présente invention a justement pour objet une nouvelle composition nettoyante pour la peau permettant notamment de remédier aux inconvénients mentionnés ci-dessus. En particulier cette composition se rince de façon remarquable et présente une texture tout à fait inhabituelle. En outre, elle est simple à appliquer et se mouille parfaitement bien sous l'eau.

De façon surprenante, la demanderesse a trouvé qu'il était possible de conférer à une composition nettoyante pour la peau un aspect de solide déformable en utilisant un agent structurant ou texturant original.

Ainsi, l'invention se rapporte à une composition nettoyante pour la peau, caractérisée en ce qu'elle contient, dans un milieu cosmétiquement acceptable contenant de l'eau, un agent structurant insoluble dans ce milieu et formé de particules solides creuses, conférant à la composition un aspect de solide déformable dans lequel le milieu est emprisonné dans l'espace interparticulaire, cet agent étant apte à s'éliminer de la peau au moyen d'un diluant et les particules étant présentes en une concentration au moins égale à la charge pigmentaire volumique critique.

Il est connu du document EP-A-486394 d'utiliser des particules solides dans des poudres coulées anhydres. Par ailleurs, le document EP-A-252463 décrit dans son exemple 7 une composition de savon broyé à base de savon et de particules renfermant un agent émollient. Toutefois, ces documents ne décrivent pas de compositions nettoyantes contenant de l'eau et des particules creuses en une concentration au moins égale à la charge pigmentaire volumique critique.

L'invention a aussi pour objet l'utilisation d'un agent structurant dans une composition nettoyante pour la peau, contenant de l'eau, pour lui conférer un aspect de solide déformable, cet agent étant formé de particules solides creuses et étant apte à être éliminé au moyen d'un diluant, les particules étant présentes en une concentration au moins égale à la charge pigmentaire volumique critique.

L'un des avantages de cette texture solide est que la composition de l'invention ne risque pas de s'échapper de son conditionnement notamment lors de son transport. Par ailleurs cette composition est très facilement préhensible et ne s'écoule pas entre les doigts. Son dosage est beaucoup plus simple que celui des liquides habituels. D'autre part, comme on peut en prendre la dose voulue lors de chaque utilisation, il ne se pose pas de problème d'usure comme pour un savon.

Selon l'invention, la composition se présente sous l'aspect d'un solide déformable, sec, ne tachant pas et ressemblant à de la guimauve (voir le document US-A-3 682 659 pour la consistance de la guimauve). Ce solide peut être modelé comme de la pâte à modeler pour enfants. Il peut être rompu facilement à la main afin de ne prélever que la quantité nécessaire de produit. En particulier, cette composition peut être conditionnée sous forme de monodose, particulièrement avantageuse sur le plan hygiènique, et par exemple sous forme de petits cubes, de billes ou de berlingots.

Grâce aux particules de l'invention, il est notamment possible d'obtenir une structure homogène (solide déformable) pour des constituants conduisant normalement à deux phases distinctes (constituants non miscibles par exemple huile/eau).

En vue d'obtenir un solide au toucher agréable et doux, il est préférable d'utiliser des particules ayant une granulométrie de 1 µm à 300 µm, par exemple de 5 µm à 200 µm et de préférence de 10 µm à 100 µm et mieux de 15 µm à 40 µm.

La grande douceur apportée par ces particules permet l'utilisation de la composition nettoyante de l'invention pour les personnes à peau sensible.

Afin de conférer à la composition de l'invention, un aspect aéré et léger, on utilise avantageusement, des particules ayant une densité inférieure à 0,09 et mieux inférieure à 0,06 et encore mieux inférieure à 0,04.

En vue d'obtenir cette faible densité, on utilise avantageusement des particules creuses remplies d'un gaz. Ce gaz peut être de l'air, de l'azote, de l'isobutane, de l'isopentane, etc.

Ces particules peuvent être réalisées en différents matériaux inertes ne réagissant pas chimiquement avec le milieu ou support cosmétiquement acceptable ; en particulier ces particules ne réagissent pas avec les huiles, les tensioactifs, l'eau et les différents autres constituants de la composition, tels que les actifs.

L'agent texturant de l'invention présente la particularité de s'éliminer facilement de la peau par simple dilution. Il joue en fait le rôle de véhicule ou de réservoir pour le support cosmétique. Il permet, en outre, de récupérer le support et notamment le ou les actifs, emprisonnés dans le solide déformable, lorsque c'est nécessaire par simple dilution à l'eau. Ceci est probablement dû au fait que le support cosmétique est logé dans les espaces interparticulaires du solide et non dans les particules.

Outre l'eau, on peut utiliser comme diluant de l'eau chargée en sels.

Comme critère de choix de l'agent texturant, on peut réaliser le test suivant :
- ajout de particules déterminées dans de l'eau contenant un colorant classiquement utilisé dans le domaine nettoyant tel que le sel disodique de bleu brillant FCF codifié dans le Color Index sous la référence Cl 42090, jusqu'à l'obtention d'une pâte colorée,
- versement d'une goutte d'eau sur la pâte.

Lorsque la pâte au point d'impact de la goutte d'eau est beaucoup plus claire que le reste de la pâte, cela signifie que les particules considérées sont candidates comme agent texturant. Inversement, lorsque la pâte au point d'impact ne s'est pas décolorée, les particules considérées ne sont nullement appropriées.

Les particules inertes sont avantageusement réalisées en matériaux thermoplastiques comme les polyamides tels que le Nylon, les polymères ou copolymères d'acrylonitrile, de chlorure de vinylidène, de chlorure de vinyle et/ou de monomère acrylique ou styrénique, éventuellement expansés. Le monomère acrylique est par exemple un acrylate ou méthacrylate de méthyle ou d'éthyle. Le monomère styrénique est par exemple l'α-méthyl-styrène ou le styrène.

De préférence, les particules sont des particules creuses déformables d'un copolymère expansé de chlorure de vinylidène et d'acrylonitrile ou de chlorure de vinylidène, d'acrylonitrile et de méthacrylate. On peut par exemple utiliser un copolymère contenant : de 0 % à 60% de motifs dérivés du chlorure de vinylidène, de 20 % à 90% de motifs dérivés d'acrylonitrile et de 0 % à 50 % de motifs dérivés d'un monomère acrylique ou styrénique, la somme des pourcentages (en poids) étant égale à 100. Ces particules se présentent notamment à l'état sec ou hydraté et peuvent être obtenues, par exemple, selon les procédés décrits dans les brevets et demandes de brevet EP-A-56 219, EP-A-348 572, EP-A-320 473, EP-A-112 807 et US-A-3 615 972.

Ces particules creuses peuvent être par exemple celles formées d'un terpolymère de chlorure de vinylidène, d'acrylonitrile et de méthacrylate, vendues sous la marque EXPANCEL par la société Nobel Casco sous les références 551 DE 12 (granulométrie d'environ 12 µm et masse volumique 40 kg/m³), 551 DE 20 (granulométrie d'environ 30 µm et masse volumique 65 kg/m³), 551 DE 50 (granulométrie d'environ 40 µm). On peut aussi citer les microsphères formées du même terpolymère expansé à l'état sec ayant une granulométrie d'environ 18 µm et une masse volumique d'environ 60 à 80 kg/m³, appelées ci-dessous EL 23, ou ayant une granulométrie d'environ 34 µm et une masse volumique d'environ 20 kg/m³, appelées ci-dessous EL 43, et ayant une granulométrie d'environ 150 µm, appelées ci-dessous EL 55.

Comme autres particules creuses polymériques utilisables dans l'invention, on peut encore citer les polymères et les copolymères obtenus à partir des esters ou acides, itaconique, citraconique, maléique, fumarique, de l'acétate ou lactate de vinyle (voir à cet effet le document JP-A-2-112304), ou encore des particules de copolymère non expansé de chlorure de vinylidène et d'acrylonitrile ou de chlorure de vinylidène, d'acrylonitrile et de méthacrylate, vendues sous la marque EXPANCEL avec la référence 551 WU.

En revanche, les particules d'amidon de maÏs, de silice pyrogénée, de polyéthylène, de polyuréthane ou de polyester non expansé ne permettent pas d'obtenir une composition solide qui s'élimine bien de la peau lors du rinçage.

Il est certes connu de modifier la viscosité d'un milieu liquide avec des particules solides (voir à ce sujet l'article Elsevier Sequoia, 1992, Progress in Organic Coatings, 21, p.255-267, de A. Toussaint "Choice of rheological model for steady flow : application to industrial concentrated suspensions") mais personne jusqu'à ce jour n'a ni décrit ni suggéré d'utiliser le produit solide, obtenu à partir d'une certaine concentration des particules, dans le domaine nettoyant pour stocker le milieu dans lequel sont dispersées les particules.

Autrement dit, l'obtention ou non du solide déformable est liée à la quantité d'agent structurant utilisée dans la composition ; au-dessus d'une certaine quantité de particules, appelée charge pigmentaire volumique critique et notée CPVC on note une augmentation brusque de la viscosité du milieu. La CPVC est fonction du milieu et de la nature des particules ; elle doit donc être déterminée à chaque fois. Sa détermination ne pose aucun problème pour l'homme du métier. On peut, par exemple, utiliser la méthode officielle ASTM pour déterminer la CPVC

L'invention a encore pour objet l'utilisation de la composition définie précédemment pour le nettoyage de la peau.

Ainsi, l'invention a encore pour objet un procédé de nettoyage de la peau, consistant à appliquer sur la peau une composition telle définie ci-dessus, puis à rincer la peau.

La composition de l'invention contient, outre les particules texturantes, tous les constituants classiquement utilisés dans les compositions nettoyantes. Ces constituants sont notamment des huiles minérales, végétales, synthétiques ou siliconées, de l'eau, des filtres, des parfums, des tensioactifs moussants et/ou nettoyants, des polymères, des conservateurs, des antioxydants, des agents régulateurs de pH, des séquestrants, des charges etc.

La composition selon l'invention peut contenir notamment un ou plusieurs tensioactifs nettoyants et/ou moussants, qui peuvent être des tensioactifs non ioniques, anioniques et/ou amphotères, et que l'on peut utiliser en une quantité allant par exemple de 5 % à 60 % en poids, et de préférence de 10 % à 40 % en poids par rapport au poids total de la composition.

Comme tensioactifs non ioniques, on peut citer par exemple les condensats d'oxydes d'alkyl phénols, les condensats d'oxyde d'éthylène, d'oxyde de propylène et d'éthylène diamine, les alkylpolyglucosides, les polyéthylène glycol glycéryl esters d'acides gras comme par exemple le Polyglyceryl-3 hydroxylauryl Ether (nom CTFA) vendu sous la dénomination CHIMEXANE NF par la Société CHIMEX.

Comme tensioactifs anioniques, on peut citer par exemple les polyalkylène glycols éther d'alcools gras comme par exemple les taurates, les acyl lactylates, les alkyl sulfates, les alkyl sulfates polyoxyéthylénés, les alkyl éthers sulfates, les alkyl éthers carboxylates, les monoalkyl ou dialkyl phosphates et les alkyl phosphate éthoxylés, les N-acyl sarcosinates, les N-acyl glutamates, les acyl iséthionates, les polysorbates et les succinamates.

Comme tensioactifs amphothères ou zwitterions, on peut citer par exemple les bétaïnes et les dérivés de bétaïnes, les sultaïnes et les dérivés de sultaïnes, les dérivés imidazolinium.

On peut aussi utiliser dans les compositions selon l'invention des tensioactifs siliconés. On peut citer comme tensioactifs siliconés les polydiméthylsiloxanes à groupements glucosides comme le SLM SPG 120 de la Société WACKER, ou encore les dérivés de polydiméthylsiloxane à groupements alkyl phosphobétaïnes telle que le PECOSIL SPB-1240 de la Société U.C.I.B.

La composition selon l'invention peut, en outre, contenir des huiles, qui peuvent être utilisées en une quantité allant de 0 % à 30 % en poids, et de préférence de 0 % à 10 % en poids par rapport au poids total de la composition.

Les huiles utilisées dans les compositions selon l'invention peuvent être choisies par exemple parmi les huiles minérales comme l'huile de paraffine et l'huile de vaseline ; les huiles d'origine animale comme le perhydrosqualène ; les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisettes, le beurre de karité et sa fraction liquide, l'huile de palme, l'huile de noyau d'abricot, l'huile de calophyllum, l'huile de son de riz, l'huile de germes de maïs, l'huile de germes de blé, l'huile de soja, l'huile de tournesol, l'huile de carthame, l'huile de passiflore et l'huile de seigle ; les huiles de synthèse telles que les esters gras comme l'huile de purcellin, le myristate de butyle, le myristate d'isopropyle, le stéarate d'hexadécyle, le stéarate d'isopropyle, le stéarate d'octyle, le stéarate d'isodécyle, l'oléate de décyle, le laurate d'héxyle, le dicaprylate de propylène glycol et les esters dérivés d'acide lanolique comme le lanolate d'isopropyle, le lanolate d'isocétyle, les isoparaffines, les acétylglycérides, les octanoates d'alcools et de polyalcools tels ceux de glycol et de glycérol, les ricinoléates d'alcools et de polyalcools, les triglycérides d'acides gras ; les huiles de silicone telles que les cyclométhicones, les polydiméthylsiloxanes volatiles et/ou non volatiles ou encore les phényl diméthyl siloxanes.

En outre, de façon connue, les compositions nettoyantes de l'invention peuvent contenir des adjuvants habituels dans le domaine du nettoyage de la peau, tels que des actifs hydrophiles ou lipophiles, des conservateurs, des antioxydants, des parfums, des colorants. Ces adjuvants sont utilisés dans les proportions habituelles des compositions nettoyantes, et par exemple de 0,01 à 10 % en poids par rapport au poids total de la composition.

On peut citer par exemple comme actifs hydrophiles ou lipophiles les agents hydratants tels que les polyols, et notamment la glycérine, les antibactériens tels que l'octopirox et le triclosan, les agents kératolytiques tels que l'acide salicylique.

Les exemples ci-après sont donnés à titre illustratif et non limitatif en vue de mieux faire ressortir les caractéristiques de l'invention.

### Exemple 1 : Composition nettoyante pour peaux grasses à tendance acnéique

(nomenclature CTFA)

| *Phase A* | |
|---|---|
| Sodium Laureth Sulfate (Texapon N70 vendu par la | |
| société Henkel) (tensioactif anionique) | 12,5 % |
| Disodium Cocoamphodiacetate (Miranol C2M Concentré vendu par la société Rhone-Poulenc) (tensioactif amphotère) | 4,3 % |
| Sodium Cocoyl isethionate de sodium/Sodium isethionate (90/10) (Gerapon AC78 vendu par la société Rhône-Poulenc) (tensioactif anionique) | 0,8 % |
| Glycérine | 3,0 % |
| Conservateur | qs |
| Eau déminéralisée | qsp 100 % |

| *Phase B* | |
|---|---|
| Fraction liquide de beurre de karité | 2,0 % |
| Octopirox (antibactérien) | 0,1 % |
| Parfum | qs |
| Acide salicylique (kératolytique) | 0,5 % |

| *Phase C* | |
|---|---|
| EXPANCEL 551 DE 20 | 14,0 % |

Le mode opératoire consiste à mélanger les différents constituants de la phase A, en chauffant si besoin, pour obtenir une phase homogène, à y ajouter la phase B, puis à homogénéiser le mélange et à y ajouter la phase C en mélangeant jusqu'à obtention d'une pâte homogène.

On obtient une pâte blanche, non collante, facilement modelable, facilement mouillable, d'application et d'élimination faciles, douce au toucher, ayant un bon pouvoir moussant.

### Exemple 2 : Composition nettoyante pour peaux grasses à tendance acnéique

(nomenclature CTFA)

| *Phase A* | |
|---|---|
| PEG-120 Methylglucose Dioleate (Glucamate DOE 120 vendu par la société Amerchol) (tensioactif non ionique) | 3,5 % |
| Sodium Lauroyl Sarcosinate (Oramix L30 vendu par la société Seppic) (tensioactif anionique) | 14,5 % |
| Disodium laureth Sulfosuccinate (Setacin 103 Special vendu par la société Zchimmer Schwarz) (tensioactif anionique) | 2,4 % |
| Lauramide DEA (Comperlan LMD vendu par la société Henkel) (tensioactif non ionique) | 2,0 % |
| Eau déminéralisée | qsp 100 % |

| *Phase B* | |
|---|---|
| Huile de germes de maïs | 2,5 % |
| Octopirox (antibactérien) | 0,2 % |
| Acide lactique | qsp pH 7 |

| *Phase C* | |
|---|---|
| EXPANCEL 551 DE 20 | 8,0 % |

Le mode opératoire est le même que pour l'exemple précédent et on obtient une pâte ayant des propriétés analogues.

### Exemple 3 : Composition nettoyante pour tout type de peaux

(nomenclature CTFA)

| *Phase A* | |
|---|---|
| Sodium Laureth Sulfate (Texapon N70 vendu par la société Henkel) (tensioactif anionique) | 14 % |
| Disodium Cocoamphodiacétate (Miranol C2M Concentré vendu par la société Rhone-Poulenc) (tensioactif amphotère) | 5 % |
| Sodium Cocoylisethionate (Hostapon SCI vendu par la société Hoescht) (tensioactif anionique) | 1 % |
| Hydrogenated Talloweth-60 Myristyl Glycol (Elfacos GT 282 S vendu par la société Akzo) (tensioactif non ionique) | 2 % |
| Eau déminéralisée | qsp 100 % |

| *Phase B* | |
|---|---|
| Fraction liquide de beurre de karité | 3 % |

| *Phase C* | |
|---|---|
| EXPANCEL 551 DE 20 | 11 % |

Le mode opératoire est le même que pour l'exemple 1 et on obtient une pâte ayant des propriétés analogues.

### Exemple 4 : Composition nettoyante pour peaux sensibles

(Nomenclature CTFA)

| *Phase A* | |
|---|---|
| Sodium Lauroyl Sarcosinate (Oramix L30 vendu par la société Seppic) (tensioactif anionique) | 7,0 % |
| Disodium laureth Sulfosuccinate (Setacin 103 Special vendu par la société Zchimmer Schwarz) (tensioactif anionique) | 2,4 % |
| Glycérine | 5,0 % |
| Eau déminéralisée | qsp 100 % |

| *Phase B* | |
|---|---|
| Huile d'amande douce | 1,0 % |

| *Phase C* | |
|---|---|
| Microsphères EL 23 | 10,5 % |

Le mode opératoire est le même que pour l'exemple 1 et on obtient une pâte ayant des propriétés analogues.

### Exemple 5 : Composition nettoyante pour tout type de peaux

(Nomenclature CTFA)

| *Phase A* | |
|---|---|
| Polyglyceryl-3 Hydroxylauryl Ether (Chimexane NF vendu par la société Chimex) (tensioactif non ionique) | 6 % |
| Sodium Laureth Sulfate (Texapon N70 vendu par la société Henkel) (tensioactif anionique) | 12 % |
| Eau déminéralisée | qsp 100 % |

| *Phase B* | |
|---|---|
| Dimethicone (Silbione Oils 70047 V 300 vendu par la société Rhône-Poulenc) (huile) | 1 % |

| *Phase C* | |
|---|---|
| EXPANCEL 551 DE 20 | 8 % |

Le mode opératoire est le même que pour l'exemple 1 et on obtient une pâte ayant des propriétés analogues.

### Exemple 6 : Composition nettoyante pour peaux très sèches

(Nomenclature CTFA)

| *Phase A* | |
|---|---|
| Polyglyceryl-3 Hydroxylauryl Ether (Chimexane NF vendu par la société Chimex) (tensioactif non ionique) | 10 % |
| Triclosan (antibactérien) | 1 % |
| Eau déminéralisée | qsp 100 % |

| *Phase B* | |
|---|---|
| Fraction liquide de beurre de karité | 2 % |
| Caprylic/capric Triglyceride | 5 % |

| *Phase C* | |
|---|---|
| Microsphères EL 23 | 8 % |

Le mode opératoire est le même que pour l'exemple 1 et on obtient une pâte ayant des propriétés analogues.

## Revendications

1. Composition nettoyante pour la peau, caractérisée en ce qu'elle contient, dans un milieu cosmétiquement acceptable contenant de l'eau, un agent structurant insoluble dans ce milieu et formé de particules solides creuses, conférant à la composition un aspect de solide déformable, le milieu étant emprisonné dans l'espace interparticulaire, cet agent étant apte à s'éliminer de la peau au moyen d'un diluant et les particules étant présentes en une concentration au moins égale à la charge pigmentaire volumique critique.

2. Composition selon la revendication 1, caractérisée en ce que les particules ont une granulométrie de 1 µm à 300 µm.

3. Composition selon la revendication 1 ou 2, caractérisée en ce que les particules ont une granulométrie de 10 µm à 100 µm.

4. Composition selon l'une des revendications précédentes, caractérisée en ce que les particules ont une densité inférieure à 0,09.

5. Composition selon l'une quelconque des revendications précédentes, caractérisée en ce que les particules ont une densité inférieure à 0,04.

6. Composition selon l'une quelconque des revendications précédentes, caractérisée en ce que les particules sont en matériau thermoplastique.

7. Composition selon l'une quelconque des revendications précédentes, caractérisée en ce que les particules sont réalisées en un matériau choisi parmi le Nylon, les polymères et copolymères de chlorure de vinylidène, d'acrylonitrile et/ou d'un monomère acrylique ou styrénique.

8. Composition selon l'une quelconque des revendications précédentes, caractérisée en ce que les particules sont des particules creuses de copolymère de chlorure de vinylidène, d'acrylonitrile et/ou d'un monomère acrylique ou styrénique, expansé.

9. Composition selon l'une quelconque des revendications précédentes, caractérisée en ce qu'elle contient, en outre, au moins un tensioactif moussant et/ou nettoyant.

10. Utilisation de la composition selon l'une quelconque des revendications précédentes pour nettoyer la peau.

11. Procédé de nettoyage de la peau, consistant à appliquer sur la peau une composition selon l'une quelconque des revendications 1 à 9, à faire mousser puis à rincer la peau.

12. Utilisation d'un agent structurant dans une composition nettoyante pour la peau, contenant de l'eau dans un milieu cosmétiquement acceptable, pour lui conférer un aspect de solide déformable, le milieu étant emprisonné dans l'espace interparticulaire, cet agent étant formé de particules solides creuses et étant apte à être éliminé au moyen d'un diluant, les particules étant présentes en une concentration au moins égale à la charge pigmentaire volumique critique.

13. Utilisation selon la revendication 12, caractérisée en ce que les particules ont une granulométrie allant de 1 µm à 300 µm.

14. Utilisation selon la revendication 12 ou 13, caractérisée en ce que les particules ont une granulométrie allant de 10 µm à 100 µm.

15. Utilisation selon l'une des revendications 12 à 13, caractérisée en ce que les particules ont une densité inférieure à 0,09.

16. Utilisation selon l'une des revendications 12 à 15, caractérisée en ce que les particules ont une densité inférieure à 0,04.

17. Utilisation selon l'une des revendications 12 à 16, caractérisée en ce que les particules sont en matériau thermoplastique.

18. Utilisation selon l'une des revendications 12 à 17, caractérisée en ce que les particules sont réalisées en un matériau choisi parmi le Nylon, les polymères et copolymères de chlorure de vinylidène, d'acrylonitrile et/ou d'un monomère acrylique ou styrénique.

19. Utilisation selon l'une des revendications 12 à 18, caractérisée en ce que les particules sont des particules creuses de copolymère de chlorure de vinylidène, d'acrylonitrile et/ou d'un monomère acrylique ou styrénique, expansé.

20. Utilisation selon l'une des revendications 12 à 19, caractérisée en ce qu'elle contient, en outre, au moins un tensioactif moussant et/ou nettoyant.

## Patentansprüche

1. Reinigende Zusammensetzung für die Haut, dadurch gekennzeichnet, daß sie in einem kosmetisch akzeptablen Medium, das Wasser enthält, ein in diesem Medium unlösliches Strukturierungsmittel enthält, das aus festen, hohlen Partikeln gebildet wird, die der Zusammensetzung das Aussehen eines verformbaren Feststoffs geben, wobei das Medium in den Zwischenräumen eingeschlossen ist, das Mittel durch ein Verdünnungsmittel von der Haut entfernt werden kann und die Partikel in einer Konzentration vorliegen, die zumindest der kritischen, volumenbezogenen Pigmentkonzentration entspricht.

2. Zusammensetzung nach Anspruch 1, dadurch gekennzeichnet, daß die Partikel eine Partikelgröße von 1 µm bis 300 µm aufweisen.

3. Zusammensetzung nach Anspruch 1 oder 2, dadurch gekennzeichnet, daß die Partikel eine Partikelgröße von 10 µm bis 100 µm aufweisen.

4. Zusammensetzung nach einem der vorhergehenden Ansprüche, dadurch gekennzeichnet, daß die Partikel eine Dichte unter 0,09 aufweisen.

5. Zusammensetzung nach einem der vorhergehenden Ansprüche, dadurch gekennzeichnet, daß die Partikel eine Dichte unter 0,04 aufweisen.

6. Zusammensetzung nach einem der vorhergehenden Ansprüche, dadurch gekennzeichnet, daß die Partikel aus einem thermoplastischen Material bestehen.

7. Zusammensetzung nach einem der vorhergehenden Ansprüche, dadurch gekennzeichnet, daß die Partikel aus einem Material hergestellt sind, das unter Nylon und Polymeren und Copolymeren von Vinylidenchlorid, Acrylnitril und/oder einem Acryl- oder Styrolmonomer ausgewählt ist.

8. Zusammensetzung nach einem der vorhergehenden Ansprüche, dadurch gekennzeichnet, daß die Partikel expandierte Hohlpartikel eines Copolymers von Vinylidenchlorid, Acrylnitril und/oder einem Acryl- oder Styrolmonomer sind.

9. Zusammensetzung nach einem der vorhergehenden Ansprüche, dadurch gekennzeichnet, daß sie ferner einen schäumenden und/oder reinigenden grenzflächenaktiven Stoff enthält.

10. Verwendung der Zusammensetzung nach einem der vorhergehenden Ansprüche zur Reinigung der Haut.

11. Verfahren zur Reinigung der Haut, das darin besteht, auf die Haut eine Zusammensetzung nach einem der Ansprüche 1 bis 9 aufzutragen, sie zum Schäumen zu bringen und dann die Haut abzuspülen.

12. Verwendung eines Strukturierungsmittels in einer reinigenden Zusammensetzung für die Haut, die in einem kosmetisch akzeptablen Medium Wasser enthält, um der Zusammensetzung das Aussehen eines verformbaren Feststoffs zu verleihen, wobei das Medium in den Zwischenräumen eingeschlossen ist und das Mittel aus festen, hohlen Partikeln gebildet wird, die durch ein Verdünnungsmittel von der Haut entfernt werden können, wobei die Partikel in einer Konzentration vorliegen, die zumindest der kritischen, volumenbezogenen Pigmentkonzentration entspricht.

13. Verwendung nach Anspruch 12, dadurch gekennzeichnet, daß die Partikel eine Partikelgröße von 1 µm bis 300 µm aufweisen.

14. Verwendung nach Anspruch 12 oder 13, dadurch gekennzeichnet, daß die Partikel eine Partikelgröße von 10 µm bis 100 µm aufweisen.

15. Verwendung nach einem der Ansprüche 12 bis 13, dadurch gekennzeichnet, daß die Partikel eine Dichte unter 0,09 aufweisen.

16. Verwendung nach einem der Ansprüche 12 bis 15, dadurch gekennzeichnet, daß die Partikel eine Dichte unter 0,04 aufweisen.

17. Verwendung nach einem der Ansprüche 12 bis 16, dadurch gekennzeichnet, daß die Partikel aus einem thermoplastischen Material bestehen.

18. Verwendung nach einem der Ansprüche 12 bis 17, dadurch gekennzeichnet, daß die Partikel aus einem Material hergestellt sind, das unter Nylon und Polymeren und Copolymeren von Vinylidenchlorid, Acrylnitril und/oder einem Acryl- oder Styrolmonomer ausgewählt ist.

19. Verwendung nach einem der Ansprüche 12 bis 18, dadurch gekennzeichnet, daß die Partikel expandierte Hohlpartikel eines Copolymers von Vinylidenchlorid, Acrylnitril und/oder einem Acryl- oder Styrolmonomer sind.

20. Verwendung nach einem der Ansprüche 12 bis 19, dadurch gekennzeichnet, daß sie ferner einen schäumenden und/oder reinigenden grenzflächenaktiven Stoff enthält.

## Claims

1. Skin cleansing composition, characterized in that it contains, in a cosmetically acceptable medium containing water, a structuring agent insoluble in this medium and formed of hollow solid particles, which imparts a deformable solid appearance to the composition, the medium being trapped in the interparticulate space, this agent being able to be removed from the skin by means of a diluent, and the particles being present in a concentration at least equal to the critical pigment charge volume.

2. Composition according to Claim 1, characterized in that the particles have a particle size of from 1 µm to 300 µm.

3. Composition according to Claim 1 or 2, characterized in that the particles have a particle size of from 10 µm to 100 µm.

4. Composition according to one of the preceding claims, characterized in that the particles have a density of less than 0.09.

5. Composition according to any one of the preceding claims, characterized in that the particles have a density of less than 0.04.

6. Composition according to any one of the preceding claims, characterized in that the particles are made of a thermoplastic material.

7. Composition according to any one of the preceding claims, characterized in that the particles are made of a material chosen from nylon, polymers and copolymers of vinylidene chloride, of acrylonitrile and/or of an acrylic or styrene monomer.

8. Composition according to any one of the preceding claims, characterized in that the particles are hollow particles of copolymer of vinylidene chloride, of acrylonitrile and/or of an acrylic or styrene monomer, which is expanded.

9. Composition according to any one of the preceding claims, characterized in that it additionally contains at least one foaming and/or cleansing surfactant.

10. Use of the composition according to any one of the preceding claims for cleansing the skin.

11. Process for cleansing the skin, consisting in applying a composition according to any one of Claims 1 to 9 to the skin, in lathering and then in rinsing the skin.

12. Use of a structuring agent in a skin cleansing composition, containing water in a cosmetically acceptable medium, in order to impart a deformable solid appearance thereto, the medium being trapped in the interparticulate space, this agent formed from hollow solid particles and being able to be removed by means of a diluent, and the particles being present in a concentration at least equal to the critical pigment charge volume.

13. Use according to Claim 12, characterized in that the particles have a particle size ranging from 1 µm to 300 µm.

14. Use according to Claim 12 or 13, characterized in that the particles have a particle size ranging from 10 µm to 100 µm.

15. Use according to one of Claims 12 to 13, characterized in that the particles have a density of less than 0.09.

16. Use according to one of Claims 12 to 15, characterized in that the particles have a density of less than 0.04.

17. Use according to one of Claims 12 to 16, characterized in that the particles are made of a thermoplastic material.

18. Use according to one of Claims 12 to 17, characterized in that the particles are made of a material chosen from nylon, polymers and copolymers of vinylidene chloride, of acrylonitrile and/or of an acrylic or styrene monomer.

19. use according to one of Claims 12 to 18, characterized in that the particles are hollow particles of copolymer of vinylidene chloride, of acrylonitrile and/or of an acrylic or styrene monomer, which is expanded.

20. Use according to one of Claims 12 to 19, characterized in that the composition additionally contains at least one foaming and/or cleansing surfactant.
